# EUROPÄISCHE PATENTSCHRIFT

(11) **EP 1 528 148 B1**
(45) Veröffentlichungstag und Bekanntmachung des Hinweises auf die Patenterteilung: **01.05.2013**
(21) Anmeldenummer: 04025244.7
(22) Anmeldetag: 22.10.2004
(51) Int. Cl.: D21C 3/22, G01N 33/34

(54) **Verfahren und Vorrichtung zur Prozessführung bei der Zellstoffkochung**
Method and apparatus for conducting the process of cooking cellulose pulp
Méthode et appareil pour conduire le procédé de cuisson de pâte cellulosique

(30) Priorität: 27.10.2003 DE 10350075
(43) Veröffentlichungstag der Anmeldung: 04.05.2005
(73) Patentinhaber: Siemens Aktiengesellschaft, 80333 München (DE)
(72) Erfinder: Gong, Jian, 80939 München (DE); Lampe, Uwe, Dr., 21614 Buxtehude (DE); Mickal, Volkmar, Dr., 91056 Erlangen (DE); Reinschke, Johannes, Dr., 90419 Nürnberg (DE)

(56) Entgegenhaltungen:
- EP-A1- 0 946 816
- WO-A2-97/10384
- DE-A1- 19 510 008
- US-A- 4 978 425
- US-A- 5 486 915

## Beschreibung

Die Erfindung betrifft ein Verfahren gemäß dem Oberbegriff des Patentanspruchs 1 sowie eine Vorrichtung gemäß dem Oberbegriff des Patentanspruchs 12.

Holz ist das Rohmaterial für die Zellstoffherstellung. Die Hauptbestandteile des Holzes sind Zellulose, Hemizellulose und Lignin. Bei der Zellstoffherstellung wird durch den chemischen Holzaufschluss Lignin von den faserigen Bestandteilen Zellulose und Hemizellulose getrennt. Dies erfolgt in der Regel durch Kochung in einem kontinuierlichen oder in einem diskontinuierlichen Prozess. Der Holzaufschluss ist ein anspruchsvoll und schwer zu handhabender Komplex chemischer und physikalischer Vorgänge.

Aus der EP 0 946 816 B1 ist ein Verfahren zur Herstellung von Zellstoff bekannt, bei dem unter Verwendung eines Zustandsmodells und/oder eines Prozessmodells Stellgrößen für ein Prozessleitsystem gebildet werden. Diese Stellgrößen können jedoch aufgrund der Komplexität der Vorgänge bei der Zellstoffherstellung nur verhältnismäßig ungenau bereitgestellt werden.

Es ist Aufgabe der Erfindung, die Prozessführung bei der Zellstoffkochung zu verbessern.

Die Aufgabe wird gelöst durch ein Verfahren der eingangs genannten Art, wobei mindestens ein dynamisches Kinetikmodell mit variablen Parametern zur Prozessführung verwendet wird, wobei das mindestens eine Kinetikmodell mit Hilfe von Modellkochungen mit unterschiedlichen Temperaturen und Konzentrationen der aktiven Kochchemikalien, variiert innerhalb der realen Kochbedingungen im Zellstoffherstellungsprozess, ermittelt wird. Durch die erfindungsgemäße Verwendung dynamischer Kinetikmodelle mit variablen Parametern werden die Vorhersagefehler der verwendeten Modelle gegenüber bekannten Modellen erheblich reduziert. Die Genauigkeit der modellbasierten Vorhersagen sowie der Prozessführung wird somit wesentlich erhöht. Gegenüber bekannten Zustandsmodellen und/oder Prozessmodellen, bei denen zur Modellbildung neuronale Netze eingesetzt werden, die trainiert werden müssen, passen sich die dynamischen Kinetikmodelle den Eigenheiten der jeweiligen Kochvorgänge wesentlich besser und schneller, d.h. mit deutlich geringerem Zeitverzug, an.

Mit Vorteil werden die variablen Parameter online durch Messungen am Herstellungsprozess ermittelt. Derart kann der Vorhersagefehler fortlaufend reduziert werden. Sehr genaue Vorhersagen des Prozessverlaufs werden so besonders effizient ermöglicht.

Mit Vorteil werden Werte variabler Parameter unter Zuhilfenahme von Spektrometern ermittelt. Derart können insbesondere chemische Konzentrationen im laufenden Herstellungsprozess und darauf basierende Vorhersagen schnell und präzise ermittelt werden.

Mit Vorteil werden Werte variabler Parameter durch Messungen an den Betriebsstoffen eines Zellstoffkochers ermittelt. Durch derartige Messungen, die in der Regel zusätzlich zu Messungen an Ein- und/oder Ausgangsstoffen des Zellstoffkochers an den Betriebsstoffen im Zellstoffkocher vorgenommen werden, kann die Vorhersagegenauigkeit nochmals gesteigert werden.

Mit Vorteil wird das Kinetikmodell unter Zuhilfenahme der variablen Parameter adaptiert.

Mit Vorteil verwendet das Kinetikmodell invariante Parameter, die offline ermittelt werden. Derart kann eine erste Grobvorhersage bereits zu Beginn des Herstellungsprozesses getroffen und dann ständig verbessert werden.

Mit Vorteil werden Eingangsdaten des Kinetikmodells unter Zuhilfenahme mindestens eines Kalibrationsmodells bereitgestellt.

Mit Vorteil werden zum Bereitstellen der Eingangsdaten für das Kalibrationsmodell Spektren im NIR-Bereich gemessen. Mit Vorteil werden zum Bereitstellen der Eingangsdaten für das Kinetikmodell Spektren im NIR-Bereich gemessen. Durch derartige Messungen werden Eingangsdaten von hoher Genauigkeit bereitgestellt.

Mit Vorteil werden zum Bereitstellen der Eingangsdaten für das Kinetikmodell Spektren im MIR-Bereich gemessen. Auf diese Weise werden komplizierte und aufwändige nasschemische Bestimmungen von Lignin und Kohlehydraten eingespart.

Mit Vorteil wird der Herstellungsprozess von Zellstoff modellprädiktiv geregelt.

Mit Vorteil wir ein schwefelfreier Kochprozess von Zellstoff geführt. Bei einem schwefelfreien Kochprozess mit Alkohol und/oder alkoholischen Verbindungen als Lösungsmittel kann die Prozessführung unter Verwendung eines dynamischen Kinetikmodells mit variablen Parametern die im Kochprozess auftretenden chemischen Konzentrationen und deren Verlauf besonders schnell und präzise vorhersagen.

Die Aufgabe wird auch gelöst durch eine Vorrichtung zur Prozessführung bei der Zellstoffkochung, die mit mindestens einem Zellstoffkocher gekoppelt ist, wobei mindestens ein Steuerungsmodul und mindestens ein Prozessmodell vorgesehen ist, und wobei das Prozessmodell mindestens ein dynamisches Kinetikmodell mit variablen Parametern aufweist.

Weitere vorteilhafte Ausgestaltungen der erfindungsgemäßen Vorrichtung finden sich in den entsprechenden Unteransprüchen. Die Vorteile der Vorrichtung ergeben sich analog zu denen des Verfahrens und aus der nachfolgenden Figurenbeschreibung.

Ausführungsbeispiele der Erfindung werden anhand der beigefügten Zeichnungen erläutert. Es zeigen:
- Figur 1: einen kontinuierlichen Zellstoffkocher,
- Figur 2: einen diskontinuierlichen Zellstoffkocher,
- Figur 3: eine mit einem Zellstoffkocher gekoppelte Vorrichtung zur Prozessführung bei der Zellstoffkochung,
- Figur 4: eine einfache Darstellung eines Prozessmodells,
- Figur 5: ein Kalibrationsmodell und ein Kinetikmodell.

Zellstoff als einer der Faserstoffe zur Papiererzeugung wird durch chemischen Aufschluss von verschiedenen Holzsorten gewonnen. Dabei wird das im Holz vorhandene Lignin durch chemische Umwandlung oder auch durch geeignete Lösungsmittel so weit herausgelöst, dass sich der verbleibende Faserverband weitgehend in Einzelfasern zerlegen lässt. Bei diesem Aufschlussprozess geht auch ein Teil der Hemizellulosen in Lösung. Der wichtigste Verfahrensschritt zur Erzeugung von Zellstoff ist der Kochprozess. Dies gilt unabhängig vom gewählten chemischen Aufschlussverfahren. Der Kochprozess legt die Qualität des erzeugten Zellstoffs maßgeblich fest. Der Kochprozess läuft bei höheren Temperaturen und höheren Drücken ab.

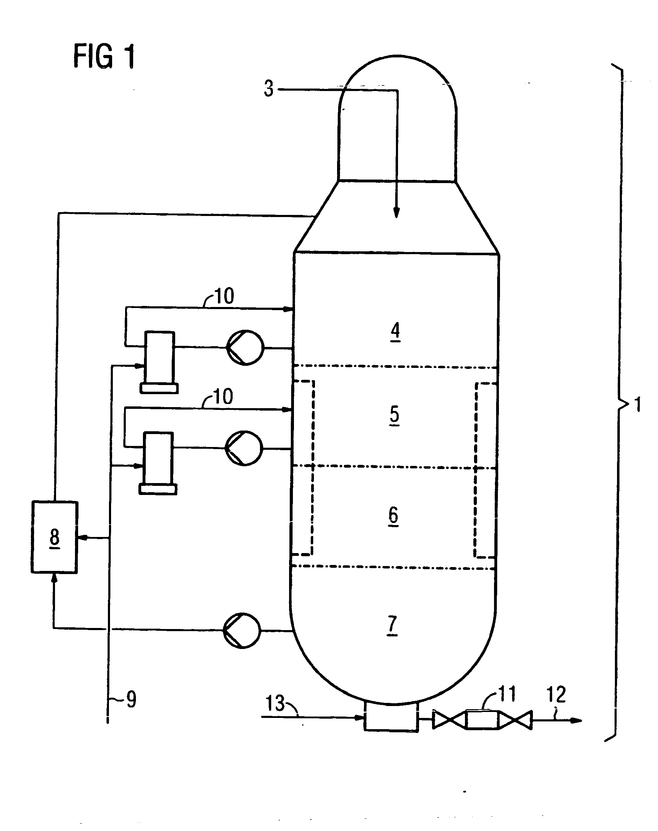

**Figur 1** zeigt einen kontinuierlichen Zellstoffkocher 1, wie er beim sogenannten kontinuierlichen Kochprozess verwendet wird. Beim kontinuierlichen Kochprozess wird Zellstoff 12 dadurch erzeugt, dass ein Rohstoff- und Chemikalienstrom kontinuierlich durch den kontinuierlichen Zellstoffkocher 1 strömt, in dem ein Umwandlungs- und Auflöseprozess stattfinden. Oft sind auch die notwendigen Waschprozesse integriert. Das kontinuierliche Verfahren eignet sich vor allem für große Produktionseinheiten mit möglichst wenig Sorten- und Rohstoffwechsel.

Der kontinuierliche Zellstoffkocher 1 kann direkt oder indirekt beheizt sein. Für den kontinuierlichen Sulfataufschluss kann ein sogenannter Kamyr-Kocher verwendet werden. Der in Figur 1 gezeigte, stehende kontinuierliche Zellstoffkocher 1 weist in seinem oberen Bereich eine Stoffzufuhr 3 auf, durch die Holzhackschnitzel, vorzugsweise zusammen mit Kochsäure, in den kontinuierlichen Zellstoffkocher 1 gefördert werden.

Der kontinuierliche Zellstoffkocher 1 lässt sich in folgende vier Zonen unterteilen: Durchtränkungszone 4, Erwärmungszone 5, Aufschlusszone 6, Diffusions-, Wasch- und Kühlzone 7.

Die Betriebsstoffe des kontinuierlichen Zellstoffkochers 1 bewegen sich durch die Schwerkraft von oben nach unten. Im Rahmen der Stoffzufuhr 3 zugeführte Holzhackschnitzel und Lauge durchlaufen so die verschiedenen heißen Zonen 4, 5, 6, 7. Der kontinuierliche Zellstoffkocher 1 weist mehrere Laugenzirkulationen 10 auf, die sowohl außerhalb des eigentlichen Kochkessels, als auch innerhalb des Kochkessels verlaufen. Die Laugenzirkulationen im Inneren des Kochkessels sind in der Zeichnung nicht näher dargestellt. Der kontinuierliche Zellstoffkocher 1 weist darüber hinaus mindestens eine Dampfleitung 9 und mindestens einen Vorwärmer 8 für die Kochlauge auf. Am unteren Ende des kontinuierlichen Zellstoffkochers 1 wird der Zellstoff 12 mit Schwarzlauge 13 verdünnt und durch eine Austragsschleuse 11 entleert.

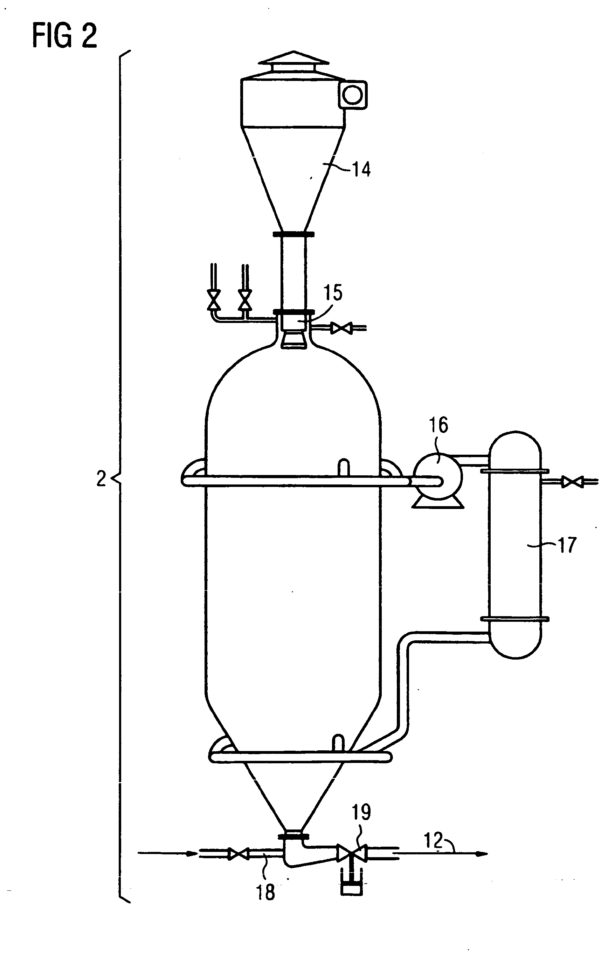

**Figur 2** zeigt einen diskontinuierlichen Zellstoffkocher 2, wie er bei einem diskontinuierlichen Kochverfahren verwendet wird. Beim diskontinuierlichen Kochverfahren wird die Gesamterzeugung von Zellstoff 12 in getrennt erzeugte Einzelchargen aufgeteilt. Zu diesem Zweck sind in der Regel mehrere diskontinuierliche Zellstoffkocher 2 vorhanden, in denen jeweils ein separater Kochprozess abläuft. Obwohl das diskontinuierliche Kochverfahren einen erhöhten Manipulations- und damit Automatisierungsaufwand erfordert, ist es immer dann von Vorteil, wenn in einer Zellstoffabrik häufige Qualitätsund/oder Rohstoffwechsel erfolgen.

Sowohl beim kontinuierlichen als auch beim diskontinuierlichen Kochprozess ist es von Vorteil, wenn das benötigte Holz in Form von Hackschnitzeln vorliegt. Die Holzhackschnitzel werden dem diskontinuierlichen Zellstoffkocher 2 von einem Holzschnitzelabscheider 14 über eine Befüllungsvorrichtung 15 zugeführt. Die Befüllungsvorrichtung 15 kann beispielsweise als mechanisches Füllgerät, als Pressluftfüllgerät oder, wie in Figur 2 angedeutet, als Dampffüllgerät ausgebildet sein. Die Beheizung des diskontinuierlichen Zellstoffkochers 2 kann direkt oder indirekt erfolgen. Der diskontinuierliche Zellstoffkocher 2 ist mit einem Wärmeaustauscher 17, einer Kochsäure-Umwälzpumpe 16 sowie einer Kochsäure-Zuführung 18 verbunden. Die Zuführung und Verteilung der Kochsäure wird durch Zwangsumwälzsysteme erreicht. Das Prinzip der Zwangsumwälzung besteht darin, dass mit Hilfe der Kochsäure-Umwälzpumpe 16 die Kochsäure an einer bestimmten Stelle des diskontinuierlichen Zellstoffkochers 2 abgezogen, aufgeheizt und an anderer Stelle in den Kocher zurückgepumpt wird, wodurch eine ständige Zirkulation der Aufschlussflüssigkeit entsteht.

Nachdem der diskontinuierliche Zellstoffkocher 2 mit Hackschnitzeln gefüllt ist, wird die Kochsäure am unteren Kocherende über eine Kochsäure-Zuführung 18 zugegeben, bis eine gute Imprägnierung der Hackschnitzel erreicht und die Luft aus dem am Kocherhals angebrachten Ventil entwichen ist. Anschließend wird Dampf zugegeben. Der Zellstoff 12 wird aufgeschlossen.

Der Verlauf der Kochung und die Qualität des produzierten Zellstoffs 12 können beispielsweise durch Änderung der Temperatur, des Druckes und der Kochzeit gesteuert werden. Nach Beendigung der Kochung und Verringerung des Drucks wird der diskontinuierliche Zellstoffkocher 2 unter Zuhilfenahme eines Entleerungsschiebers 19 entleert.

Zu den Parametern, die den Verlauf der Kochung beeinflussen, gehören auch die Imprägnierung der Hackschnitzel und insbesondere die Chemikalienzusammensetzung und -konzentrationen. Durch Veränderung dieser Parameter sowie Veränderung von Kochtemperatur und Druck kann der Verlauf der Kochung gesteuert und beeinflusst werden.

Die selben und weitere Faktoren beeinflussen auch den Kochprozess bei der kontinuierlichen Kochung. Veränderliche Parameter bei der Kochung sind insbesondere Temperatur und Durchflussgeschwindigkeiten. Bei kontinuierlichen Kochungen können höhere Temperaturen angewendet werden, da die Kochdauer wesentlich kürzer ist. Die notwendige Kochdauer wird u.a. durch das Hydromodul, den Chemikalieneinsatz, Imprägnierung und Eigenschaften der Hackschnitzel, wie z.B. Holzart und Abmessung, beeinflusst.

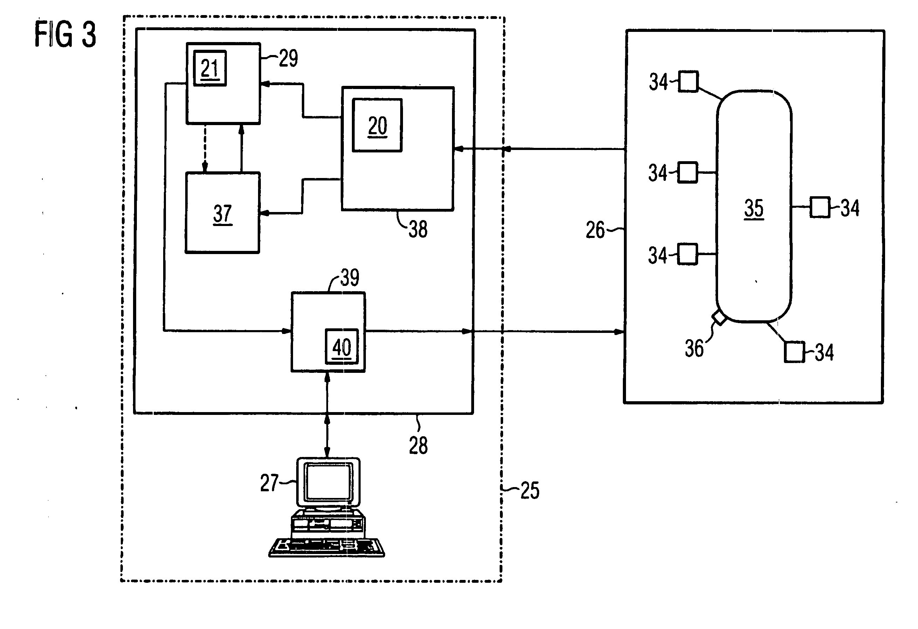

**Figur 3** zeigt eine mit einem Zellstoffkocher 35 gekoppelte Vorrichtung zur Prozessführung 25 bei der Zellstoffkochung.

Im Zellstoffkocher 35 kann beispielsweise ein Sulfatprozess, ein Sulfitprozess, oder ein IDE-Prozess (Imprägnierungs-Delignifikations- und Extraktionsprozess) oder ein Teil eines derartigen Prozesses geführt werden. Die Verwendung des erfindungsgemäßen Verfahrens bzw. der erfindungsgemäßen Vorrichtung ist besonders geeignet beim IDE-Prozess und anderen schwefelfreien Prozessen mit Alkohol und/oder alkoholischen Verbindungen als Lösungsmittel.

Der Zellstoffkocher 35 ist Bestandteil einer Anlage zur Zellstoffherstellung 26. Die Anlage zur Zellstoffherstellung 26 wird von der Vorrichtung zur Prozessführung 25 beobachtet, gesteuert und/oder geregelt. Die Vorrichtung zur Prozessführung 25 weist vorzugsweise eine Ein- und Ausgabevorrichtung 27, beispielsweise einem Bedienrechner mit Anzeigeeinheit, und/oder kann mit einer eine Ein- und Ausgabevorrichtung 27 gekoppelt. Letzteres ist in der Zeichnung nicht näher dargestellt. Die Vorrichtung zur Prozessführung 25 weist ein Steuerungsmodul 28 auf.

Das Steuerungsmodul 28 weist ein Prozessmodell 29 auf, das ausgangsseitig vorzugsweise mit einer Stellgliedberechnungseinheit 39 gekoppelt ist. Die Stellgliedberechnungseinheit 39 weist vorzugsweise eine Optimierungseinheit 40 auf. Der Stellgliedberechnungseinheit 39 werden beispielsweise unter Verwendung der ein Ein- und Ausgabevorrichtung 27 Sollwerte zugeführt. Die Stellgliedberechnungseinheit 39 gibt vorzugsweise Signale an Stellglieder des Zellstoffkochers 35.

Das Prozessmodell 29 weist ein Kinetikmodell 21 auf und ist mit einem Adaptionsmodul 37 gekoppelt. Das Steuerungsmodul 28 weist ein Modul zur Messwertverarbeitung 38 auf, dem Daten, Messwerte und Spektren von der Anlage zur Zellstoffherstellung 26, insbesondere dem Zellstoffkocher zugeführt werden. Das Modul zur Messwertverarbeitung 38 weist vorzugsweise ein Kalibrationsmodell 20 auf. Das Modul zur Messwertverarbeitung 38 gibt Daten an das Prozessmodell 29 und an das Adaptionsmodul 37.

Der Zellstoffkocher 35 und gegebenenfalls weitere Bestandteile der Anlage zur Zellstoffherstellung 26 sind mit Messvorrichtungen 36 gekoppelt. Mit Hilfe der Messvorrichtungen 36 können z.B. physikalische Größen wie Temperatur bzw. Druck am Zellstoffkocher 35 gemessen und der Vorrichtung zur Prozessführung 25 zugeführt werden.

Der Zellstoffkocher 35 weist Spektrometer 34 auf, die Spektren, vorzugsweise Transmissionsspektren, an den Betriebsstoffen des Zellstoffkochers 35 erfassen. Die Messwerte und/oder Spektren der in der Anlage zur Zellstoffherstellung 26 angeordneten Messvorrichtungen 36 bzw. Spektrometer 34 werden vorzugsweise einer Datenaufbereitung unterzogen und der Vorrichtung zur Prozessführung 25 zugeführt. Eine Datenaufbereitung kann auch in der Vorrichtung zur Prozessführung 25 erfolgen.

Das in der Vorrichtung zur Prozessführung 25 angeordnete Steuerungsmodul 28 ist den Herstellungsprozess überwachend, steuernd und/oder regelnd ausgebildet. Mittels des Prozessmodells 29 werden Daten und Vorhersagen über den laufenden Herstellungsprozess in der Anlage zur Zellstoffherstellung 26 und insbesondere im Zellstoffkocher 35 gewonnen. Die Daten und Vorhersagen werden an die Stellgliedberechnungseinheit 39 und/oder an die Ein- und Ausgabevorrichtung 27 übermittelt.

Zwischen der Anlage zur Zellstoffherstellung 26 und der Vorrichtung zur Prozessführung 25 erfolgt ein Datenfluss. Dabei können Daten mittelbar und/oder unmittelbar von und zu den Bestandteilen der Vorrichtung zur Prozessführung 25, wie beispielsweise dem Steuerungsmodul 28, dem Modul zur Messwertverarbeitung 38, der Stellgliedberechnungseinheit 39, und/oder der Ein- und Ausgabevorrichtung 27 übertragen werden.

Für die Verfolgung der Konzentrationen im Zellstoffkocher 35 müssen die Eigenschaften der Infrarot-Spektroskopie berücksichtigt werden. Mit Hilfe der mittleren Infrarot-Spektroskopie (MIR-Spektroskopie) können Fundamentalschwingungen der Moleküle mit klar unterscheidbaren Kennlinien für unterschiedliche Verbindungen beobachtet werden. Mit der nahen Infrarot-Spektroskopie (NIR-Spektroskopie) können hingegen nur gekoppelte Schwingungen verschiedener Atomgruppen und harmonische Schwingungen von Einzelverbindungen beobachtet werden. Daher werden bei der NIR-Spektroskopie Referenzmessungen der chemischen Konzentrationen und Modelle zur Korrelierung der NIR-Spektren mit den Referenzkonzentrationen benötigt.

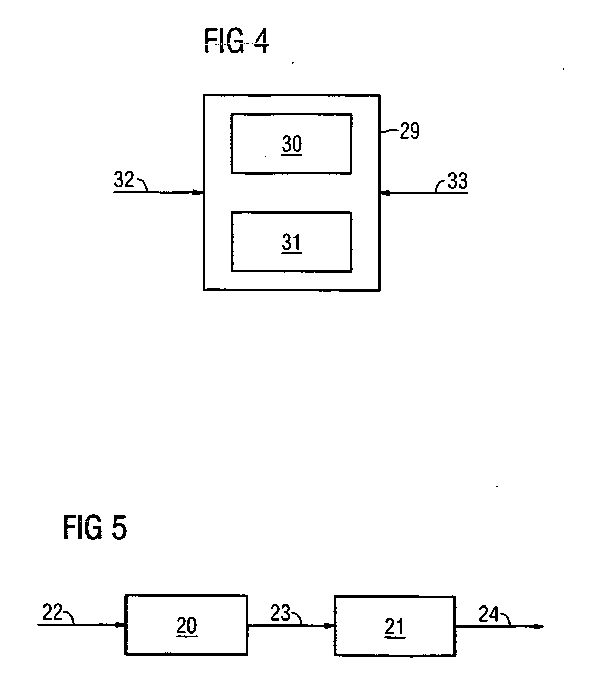

**Figur 4** zeigt das Prozessmodell 29, dem sowohl invariante Parameter 32 als auch (zeitlich) variable Parameter 33 zugeführt werden. Die Ausgänge bzw. Ausgangsgrößen des Prozessmodells 29 sind in Figur 4 nicht näher dargestellt. Das Prozessmodell 29 weist mindestens ein Kinetikmodell 30, 31 auf. Gegebenenfalls zusätzliche vorhandene Modelle sind in Figur 4 nicht näher dargestellt. Das Prozessmodell 29 weist ein Kinetikmodell für den Verbrauch von Alkali NaOH 30 und ein Kinetikmodell für die Delignifizierung und die Kohlehydrat-Degradation 31 auf. Die beiden Kinetikmodelle 30, 31 bilden die Basis für eine Online-Vorhersage von Konzentrationen in der Kochlauge. Die Vorhersage der Konzentrationsverläufe und gegebenenfalls weiterer Prozess relevanter Daten ist von Anfang an nach einigen Messpunkten möglich. Die Genauigkeit der Vorhersagen wird durch eine Online-Identifikation und -Modelladaption mit steigender Messpunktezahl verbessert.

Die Kinetikmodelle 30, 31 werden zuerst mit Hilfe von Modellkochungen mit unterschiedlichen Temperaturen und Konzentrationen der aktiven Kochchemikalien, variiert innerhalb der realen Kochbedingungen im Zellstoffherstellungsprozess, ermittelt und mit reaktionsspezifischen Aktivierungsenergien und Reaktionsordnungen als Ausgangsmodelle für die reale Kochung verwandt. Die Kinetikmodelle 30, 31 werden dann anhand anfänglich online gemessener Konzentrationswerte auf die Startbedingungen jeder Kochung angepasst. Die angepassten Kinetikmodelle 30, 31 für die jeweilige Kochung ermöglichen bereits eine exakte Vorhersage, werden jedoch durch laufende Messungen ständig verbessert.

Das Prozessmodell 29 kann zur online Vorhersage beispielsweise derart verwendet werden, dass für den kontinuierlichen Zellstoffkocher 1 eine Sollgeschwindigkeit ermittelt wird. Beim diskontinuierlichen Zellstoffkocher 2 wird beispielsweise die Abbruchzeit ermittelt. Die mit Hilfe des Prozessmodells 29 ermittelten Daten werden der Stellgliedberechnungseinheit 39 (siehe Figur 3) und/oder der Ein- und Ausgabevorrichtung 27 zugeführt.

Das Prozessmodell 29 rechnet in einem vorgegebenen Zeitschritt online Daten für einen festgelegten Zeithorizont ("Prädikationshorizont") voraus. Nach Ablauf dieses Zeitschritts werden neue variable Parameter 33 erfasst und die im Prozessmodell 29 implementierten Modelle online entsprechend angepasst. Unter Zuhilfenahme der Stellgliedberechnungseinheit 39 werden Stellwerte für einen Stellhorizont von einem oder mehreren wenigen Zeitschritten berechnet. Anschließend werden erneut Daten für den festgelegten Prädikationshorizont vorausberechnet, jedoch mit dem aktuellen Zeitpunkt als Anfangszeit. Die so implementierte Regelung fällt in die Klasse der modellprädiktiven Regelungen.

Die Kinetikmodelle 30, 31 verarbeiten invariante Parameter 32, die vorzugsweise vor Beginn des Herstellungsprozesses offline bestimmt werden. Invariante Parameter 32 umfassen beispielsweise Aktivierungsenergien, Reaktionskonstanten und Reaktionsordnungen.

Variable Parameter 33 werden den Kinetikmodellen 30, 31 während des laufenden Herstellungsprozesses zugeführt. Die variablen Parameter 33 werden vorzugsweise online bestimmt bzw. identifiziert und gegebenenfalls zusätzlich einer Adaption unterzogen. Die variablen Parameter 33 ändern sich während des Herstellungsprozesses.

Figur 5 zeigt ein einem Kinetikmodell 21 vorgeordnetes Kalibrationsmodell 20. Dem Kalibrationsmodell 20 werden Spektraldaten 22 aus NIR-Messungen zugeführt. Das Kalibrationsmodell 20 gibt Modellparameter 23 an das nachgeordnete Kinetikmodell 21 weiter. Das Kinetikmodell 21 ermittelt Vorhersagedaten 24 beispielsweise für die aktuellen und zukünftigen Konzentrationen der relevanten Kochlaugenkonzentrationen.

Invariante Parameter 32 werden dabei offline, vorzugsweise mit Hilfe von MIR-Messungen ermittelt. Mittels der offline NIR- und MIR-Messungen an ein und der selben Probe werden die Kalibrationsmodelle 20 identifiziert. Offline Messungen können dabei im Labor und/oder direkt am Zellstoffkocher 35 durchgeführt werden. Die offline Messungen werden jeweils vor der Initialisierung der Modelle 20, 21 durchgeführt, für die sie verwendet werden.

Die online durchgeführten NIR-Messungen werden mit Hilfe der Kalibrationsmodelle 20 mit MIR-Messungen korreliert. Für die MIR-Messungen bedient man sich beispielsweise der Methode der abgeschwächten Totalreflektion (ATR-Technik, "Attenuated Total Reflection").

Bei der Ermittlung der (zeitlich) invarianten Parameter 32 in den Kinetikmodellen 21 werden die Konzentrationen der Abbauprodukte von Lignin und Kohlehydraten in Kochlaugenproben, die das Fortschreiten des Prozesses widerspiegeln, vorzugsweise durch MIR-Messungen bestimmt. Dabei werden die Flächen der spezifischen Absorptionsbande der charakteristischen Bindungsarten von Lignin und Kohlehydraten benutzt. Diese dienen gleichzeitig als Referenzdaten für die Kalibrationsmodelle 20 der online-NIR-Messungen. Auf diese Weise werden die komplizierten und aufwändigen nasschemischen Bestimmungen von Lignin und Kohlehydratkonzentrationen eingespart.

Für die Online-NIR-Messungen werden die MIR-Messungen als Referenzdaten für die Erstellung der Kalibrationsmodelle 20 für Messgrößen wie Konzentrationen von Lignin und Kohlehydraten benötigt. Bei Kinetikmodellen 21, insbesondere Kinetikmodellen für die Delignifizierung und Kohlehydrat-Degradation 31, die auf gleicher Datenbasis, nämlich den spezifischen Absorptionsflächen von Lignin und Kohlehydraten, aus MIR-Spektren erstellt sind, können die online-NIR-Messdaten beispielsweise durch Translation mit den Kalibrationsmodellen 20 in die Kinetikmodelle 21 eingesetzt werden.

Zur Spektrenmessung werden vorzugsweise Sonden in der Kochlauge im Zellstoffkocher 1, 2, 35, die durch Glasfasern an ein NIR-Spektrometer gekoppelt sind, verwendet. Um die Kinetik der im Zellstoffkocher 1, 2, 35 stattfindenden Reaktionen zu bestimmen, werden die zeitlichen Verläufe im Zellstoffkocher 35 ermittelt.

Die Kalibrationsmodelle 20 können durch Ermittlung der Korrelation von NIR-Spektren mit den an den MIR-Messungen gewonnenen Referenzdaten mittels chemometrischer Methoden (z.B. PLS, PCR, neuronale Netze, usw.) erstellt werden. Dabei wird vorzugsweise in drei Schritten vorgegangen: Datenvorverarbeitung, Modellerstellung und Validierung.

Zur Online-Parameter-Identifikation wird im Kern ein überbestimmtes System linearer algebraischer Gleichungen verwendet, das im Sinne der Methode der kleinsten Quadrate gelöst wird. Dem geht eine Datenvorverarbeitung voraus, die in der Regel nichtlinear ist. Vorzugsweise werden Messwerte bzw. Konzentrationen zu diskreten Zeiten verwendet. Zur Lösung des Systems linearer Gleichungen wird vorzugsweise eine sequentielle Methode mit Hilfe einer Zerlegung in Hessenberg-Matrizen verwendet wird.

Der Grundgedanke der Erfindung lässt sich im wesentlichen wie folgt zusammenfassen:

Die Erfindung betrifft ein Verfahren zur Prozessführung bei der Zellstoffkochung 12 unter Verwendung mindestens eines Modells, wobei mindestens ein dynamisches Kinetikmodell 21, 30, 31 mit variablen Parametern 33 verwendet wird.

Die Erfindung betrifft auch eine Vorrichtung zur Prozessführung 25 bei der Zellstoffkochung, die mit mindestens einem Zellstoffkocher 35 gekoppelt ist, wobei mindestens ein Steuerungsmodul 28 und mindestens ein Prozessmodell 29 vorgesehen ist, und das Prozessmodell 29 mindestens ein dynamisches Kinetikmodell 21, 30, 31 mit variablen Parametern 33 aufweist.

Zur online Überwachung des Herstellungsprozesses von Zellstoff 12 werden Infrarot-Spektren gemessen. Online werden vorzugsweise NIR-Spektren gemessen. Grundlage bilden MIR-Messungen, die offline durchgeführt werden. Spektren werden mittels Spektrometer 34 an den flüssigen Betriebsstoffen eines Zellstoffkochers 35 gemessen.

Die MIR- Messungen dienen als Referenz für die Konzentrationen von gelöstem Lignin und Kohlehydraten. Vorzugsweise können so nasschemische Messungen von verschiedenen Konzentrationen in einer Probe durch eine einzige MIR-Messung ersetzt werden. Mit Hilfe von Referenzmessungen können invariante Parameter 32 von Kinetikmodellen 21, 20, 31 identifiziert werden. Variable Parameter 33 werden in den Kinetikmodellen 21, 20, 31 zur genauen Prozessüberwachung online mit Hilfe von am laufenden Prozess durchgeführten Messungen, vorzugsweise im NIR-Spektrum, identifiziert. Die Kinetikmodelle 21, 20, 31 ermöglichen eine sehr genaue Vorhersage des Prozessverhaltens.

## Patentansprüche

1. Verfahren zur Prozessführung bei der Zellstoffkochung (12) unter Verwendung mindestens eines Modells,
**dadurch gekennzeichnet, dass** mindestens ein dynamisches Kinetikmodell (21, 30, 31) mit variablen Parametern (33) verwendet wird, wobei das mindestens eine Kinetikmodell mit Hilfe von Modellkochungen mit unterschiedlichen Temperaturen und Konzentrationen der aktiven Kochchemikalien, variiert innerhalb der realen Kochbedingungen im Zellstoffherstellungsprozess, ermittelt wird.

2. Verfahren nach Patentanspruch 1,
**dadurch gekennzeichnet, dass** die variablen Parameter (33) online durch Messungen am Herstellungsprozess identifiziert werden.

3. Verfahren nach einem der vorangehenden Patentansprüche,
**dadurch gekennzeichnet, dass** Werte variabler Parameter (33) unter Zuhilfenahme von Spektrometern (34) ermittelt werden.

4. Verfahren nach einem der Patentansprüche 2 oder 3,
**dadurch gekennzeichnet, dass** Werte variabler Parameter (33) durch Messungen an den Betriebsstoffen eines Zellstoffkochers (35) ermittelt werden.

5. Verfahren nach einem der vorangehenden Patentansprüche,
**dadurch gekennzeichnet, dass** das Kinetikmodell (21, 30, 31) unter Zuhilfenahme der variablen Parameter (33) adaptiert wird.

6. Verfahren nach einem der vorangehenden Patentansprüche,
**dadurch gekennzeichnet, dass** das Kinetikmodell (21, 30, 31) invariante Parameter (34) verwendet, die offline ermittelt werden.

7. Verfahren nach einem der vorangehenden Patentansprüche,
**dadurch gekennzeichnet, dass** Eingangsdaten des Kinetikmodells (21, 30, 31) unter Zuhilfenahme mindestens eines Kalibrationsmodells (20) bereitgestellt werden.

8. Verfahren nach Patentanspruch 7,
**dadurch gekennzeichnet, dass** zum Bereitstellen der Eingangsdaten für das Kalibrationsmodell (20) Spektren im NIR-Bereich gemessen werden.

9. Verfahren nach einem der vorangehenden Patentansprüche,
**dadurch gekennzeichnet, dass** zum Bereitstellen der Eingangsdaten für das Kinetikmodell (21, 30, 31) Spektren im NIR-Bereich gemessen werden.

10. Verfahren nach einem der vorangehenden Patentansprüche,
**dadurch gekennzeichnet, dass** zum Bereitstellen der Eingangsdaten für das Kinetikmodell (21, 30, 31) Spektren im MIR-Bereich gemessen werden.

11. Verfahren nach einem der vorangehenden Patentansprüche,
**dadurch gekennzeichnet, dass** der Herstellungsprozess modellprädiktiv geregelt wird.

12. Verfahren nach einem der vorangehenden Patentansprüche,
**dadurch gekennzeichnet, dass** ein Schwefel freier Kochprozess geführt wird.

13. Vorrichtung zur Prozessführung (25) bei der Zellstoffkochung, die mit mindestens einem Zellstoffkocher (35) gekoppelt ist,
**dadurch gekennzeichnet, dass** mindestens ein Steuerungsmödul (28) und mindestens ein Prozessmodell (29) vorgesehen ist, und dass das Prozessmodell (29) mindestens ein dynamisches Kinetikmodell (21, 30, 31) mit variablen Parametern (33) aufweist, wobei das mindestens eine Kinetikmodell mit Hilfe von Modellkochungen mit unterschiedlichen Temperaturen und Konzentrationen der aktiven Kochchemikalien, variiert innerhalb der realen Kochbedingungen im Zellstoffherstellungsprozess, ermittelt wird.

14. Vorrichtung nach Patentanspruch 13,
**dadurch gekennzeichnet, dass** sie Mittel zur Durchführung eines Verfahrens gemäß einem der Patentansprüche 1 bis 11 aufweist.

15. Vorrichtung nach Patentanspruch 13 oder 14,
**dadurch gekennzeichnet, dass** sie mit mindestens einer Messvorrichtung (36) zur online Erfassung von Werten variabler Parameter (33) gekoppelt ist.

16. Vorrichtung nach einem der Patentansprüche 13 bis 15,
**dadurch gekennzeichnet, dass** die Vorrichtung zur Prozessführung (25) mit mindestens einem Spektrometer (34) gekoppelt ist, mit Hilfe dessen Eingangsdaten für das Kinetikmodell (21,30,31) erfasst werden.

17. Vorrichtung nach einem der Patentansprüche 13 bis 16,
**dadurch gekennzeichnet, dass** das Spektrometer (34) zur Erfassung von Werten für variable und/oder invariante Parameter (32, 33) mit dem Zellstoffkocher (35) verbunden ist.

## Claims

1. Method for process control during pulp cooking (12) using at least one model,
**characterised in that** at least one dynamic kinetic model (21, 30, 31) with variable parameters (33) is used, wherein the at least one kinetic model is determined using model cooking with different temperatures and concentrations of the active cooking chemicals, varied within the actual cooking conditions in the pulp manufacturing process.

2. Method according to claim 1,
**characterised in that** the variable parameters (33) are identified online by measurements of the manufacturing process.

3. Method according to one of the preceding claims,
**characterised in that** values of variable parameters (33) are determined with the assistance of spectrometers (34).

4. Method according to one of claims 2 or 3,
**characterised in that** values of variable parameters (33) are determined by measurements of the operating materials of a digester (35).

5. Method according to one of the preceding claims,
**characterised in that** the kinetic model (21, 30, 31) is adapted with the assistance of the variable parameters (33).

6. Method according to one of the preceding claims,
**characterised in that** the kinetic model (21, 30, 31) uses invariant parameters (34) which are determined offline.

7. Method according to one of the preceding claims,
**characterised in that** input data for the kinetic model (21, 30, 31) is provided with the assistance of at least one calibration model (20).

8. Method according to claim 7,
**characterised in that** spectra are measured in the NIR range for the provision of input data for the calibration model (20).

9. Method according to one of the preceding claims,
**characterised in that** spectra are measured in the NIR range for the provision of input data for the kinetic model (21, 30, 31).

10. Method according to one of the preceding claims,
**characterised in that** spectra are measured in the MIR range for the provision of input data for the kinetic model (21, 30, 31).

11. Method according to one of the preceding claims,
**characterised in that** the manufacturing process is governed on a model-predictive basis.

12. Method according to one of the preceding claims,
**characterised in that** a sulphur-free cooking process is performed.

13. Apparatus for process control (25) during pulp cooking, which is coupled to at least one digester (35),
**characterised in that** at least one control module (28) and at least one process model (29) is provided, and that the process model (29) has at least one dynamic kinetic model (21, 30, 31) with variable parameters (33), wherein the at least one kinetic model is determined using model cooking with different temperatures and concentrations of the active cooking chemicals, varied within the actual cooking conditions in the pulp manufacturing process.

14. Apparatus according to claim 13,
**characterised in that** it has means for carrying out a method according to one of claims 1 to 11.

15. Apparatus according to claim 13 or 14,
**characterised in that** it is coupled to at least one measurement apparatus (36) for the online determination of values of variable parameters (33).

16. Apparatus according to one of claims 13 to 15,
**characterised in that** the apparatus for process control (25) is coupled to at least one spectrometer (34), with the help of which input data for the kinetic model (21, 30, 31) is determined.

17. Apparatus according to one of claims 13 to 16,
**characterised in that** the spectrometer (34) for determining values for variable and/or invariant parameters (32, 33) is connected to the digester (35).

## Revendications

1. Procédé de conduite du procédé de cuisson ( 12 ) de pâte cellulosique, en utilisant au moins un modèle,
**caractérisé en ce qu'**on utilise au moins un modèle ( 21, 30, 31 ) de cinétique dynamique ayant des paramètres ( 33 ) variables, le au moins un modèle de cinétique étant déterminé à l'aide de cuissons modèles ayant des températures et des concentrations différentes des produits chimiques actifs de cuisson, qui se modifient dans les conditions réelles de cuisson du processus de production de pâte cellulosique.

2. Procédé suivant la revendication 1,
**caractérisé en ce qu'**on identifie les paramètres ( 33 ) variables en ligne par des mesures sur le processus de production.

3. Procédé suivant l'une des revendications précédentes,
**caractérisé en ce qu'**on détermine des valeurs des paramètres ( 33 ) variables en s'aidant de spectromètres ( 34 ).

4. Procédé suivant l'une des revendications 2 ou 3,
**caractérisé en ce qu'**on détermine des valeurs de paramètres ( 33 ) variables par des mesures sur des substances de fonctionnement d'un cuiseur ( 35 ) de pâte cellulosique.

5. Procédé suivant l'une des revendications précédentes,
**caractérisé en ce qu'**on adapte le modèle ( 21, 30, 31 ) de cinétique en s'aidant des paramètres ( 33 ) variables.

6. Procédé suivant l'une des revendications précédentes,
**caractérisé en ce que** le modèle ( 21, 30, 31 ) de cinétique utilise des paramètres ( 34 ) invariants qui sont déterminés hors ligne.

7. Procédé suivant l'une des revendications précédentes,
**caractérisé en ce qu'**on met à disposition des données d'entrée du modèle ( 21, 30, 31 ) de cinétique en s'aidant d'au moins un modèle ( 20 ) d'étalonnage.

8. Procédé suivant la revendication 7,
**caractérisé en ce que**, pour la mise à disposition des données d'entrée pour le modèle ( 20 ) d'étalonnage, on mesure des spectres dans le domaine NIR.

9. Procédé suivant l'une des revendications précédentes,
**caractérisé en ce que**, pour la mise à disposition des données d'entrée pour le modèle ( 21, 30, 31 ) de cinétique, on mesure des spectres dans le domaine NIR.

10. Procédé suivant l'une des revendications précédentes,
**caractérisé en ce que**, pour la mise à disposition des données d'entrée pour le modèle ( 21, 30, 31 ) de cinétique, on utilise des spectres dans le domaine MIR.

11. Procédé suivant l'une des revendications précédentes,
**caractérisé en ce qu'**on régule, d'une manière prédictive par modèle, le processus de production.

12. Procédé suivant l'une des revendications précédentes,
**caractérisé en ce qu'**on conduit un processus de cuisson sans soufre.

13. Dispositif de conduite ( 25 ) du processus de cuisson de pâte cellulosique, qui est couplé à au moins un cuiseur ( 35 ) de pâte cellulosique,
**caractérisé en ce qu'**il est prévu un module ( 28 ) de commande et au moins un modèle ( 29 ) de processus, et **en ce que** le modèle ( 29 ) de processus a au moins un modèle ( 21, 30, 31 ) de cinétique dynamique ayant des paramètres ( 33 ) variables, le au moins un modèle de cinétique étant déterminé avec des températures et des concentrations différentes des produits chimiques actifs de cuisson, qui se modifient dans les conditions réelles de cuisson dans le processus de production de la pâte cellulosique.

14. Dispositif suivant la revendication 13,
**caractérisé en ce qu'**il a des moyens pour effectuer un procédé suivant l'une des revendications 1 à 11.

15. Dispositif suivant la revendication 13 ou 14,
**caractérisé en ce qu'**il est couplé à au moins un dispositif ( 36 ) de mesure pour la détection en ligne de valeurs de paramètres ( 33 ) variables.

16. Dispositif suivant l'une des revendications 13 à 15,
**caractérisé en ce que** le dispositif de conduite ( 25 ) du processus est couplé à au moins un spectromètre ( 34 ), à l'aide duquel des données d'entrée pour le modèle ( 21, 30, 31 ) de cinétique sont relevées.

17. Dispositif suivant l'une des revendications 13 à 16,
**caractérisé en ce que** le spectromètre ( 34 ) pour relever des valeurs de paramètres ( 32, 33 ) variables et/ou invariants est relié au cuiseur ( 35 ) de pâte cellulosique.
